# EUROPEAN PATENT SPECIFICATION

(11) **EP 3 434 285 B1**
(45) Date of publication and mention of the grant of the patent: **30.06.2021**
(21) Application number: 17769488.2
(22) Date of filing: 24.03.2017
(51) Int. Cl.: A61K 31/575, A61K 31/397, A61K 45/06, A61P 1/16, A61P 3/06, A61P 3/10, A61P 35/00, A61P 43/00, A61P 9/00

(54) **COMBINATIONS OF OBETICHOLIC ACID AND EZETIMIBE FOR THE TREATMENT OF NAFLD AND NASH**
ZUSAMMENSETZUNGEN AUS OBETICHOLSÄURE UND EZETIMIB ZUR BEHANDLUNG VON NAFLD UND NASH
COMBINAISONS DE L'ACIDE OBÉTICHOLIQUE ET DE L'EZETIMIBE DESTINÉES AU TRAITEMENT DE LA NAFLD ET DE LA NASH

(30) Priority: 24.03.2016 CN 201610173317
(43) Date of publication of application: 30.01.2019
(73) Proprietor: Waterstone Pharmaceuticals (Wuhan) Co., Ltd., Wuhan, Hubei 430075 (CN)
(72) Inventor: LIU, Xiaoyu, Wuhan Hubei 430075 (CN); ZHANG, Faming, Wuhan Hubei 430075 (CN); CUI, Jian, Wuhan Hubei 430075 (CN); ZHANG, Sihan, Wuhan Hubei 430075 (CN); WANG, Huojian, Wuhan Hubei 430075 (CN); MENG, Yuan, Wuhan Hubei 430075 (CN); LENG, Zhengwen, Wuhan Hubei 430075 (CN); QIAN, Lina, Wuhan Hubei 430075 (CN)
(74) Representative: Maiwald Patent- und Rechtsanwaltsgesellschaft mbH
(86) International application number: PCT/CN2017/078183
(87) International publication number: WO 2017/162211

(56) References cited:
- CN-A- 102 451 161
- DATABASE WPI Week 201330 Thomson Scientific, London, GB; AN 2013-B77345 XP002794127, -& RU 2 473 342 C1 (CHERKASHOVA E A) 27 January 2013 (2013-01-27)
- DATABASE WPI Week 201682 Thomson Scientific, London, GB; AN 2016-47093V XP002794128, -& CN 105 748 487 A (WATERSTONE BIOMEDICAL TECHNOLOGY WUHAN) 13 July 2016 (2016-07-13)
- SUNDER MUDALIAR ET AL: "Efficacy and Safety of the Farnesoid X Receptor Agonist Obeticholic Acid in Patients With Type 2 Diabetes and Nonalcoholic Fatty Liver Disease", GASTROENTEROLOGY, vol. 145, no. 3, 1 September 2013 (2013-09-01), pages 574-582.e1, XP055086786, ISSN: 0016-5085, DOI: 10.1053/j.gastro.2013.05.042
- YUMIE TAKESHITA ET AL: "The effects of ezetimibe on non-alcoholic fatty liver disease and glucose metabolism: a randomised controlled trial", DIABETOLOGIA, vol. 57, no. 5, 10 January 2014 (2014-01-10), pages 878-890, XP055620521, BERLIN, DE ISSN: 0012-186X, DOI: 10.1007/s00125-013-3149-9
- LI , PEIFENG: "Studies on Mechanism of Antiasthmatic and Anti-Inflammatory Actions of CDCA", CHINA JOURNAL OF CHINESE MATERIA MEDICA, vol. 29, no. 4, 30 April 2004 (2004-04-30) , pages 349-352, XP009512534, ISSN: 1001-5302
- LIAO, XIANHUA et al.: "Advances and Prognosis in the Treatment of Non-Alcoholic Fatty Liver Disease", Ganzang = CHINESE HEPATOLOGY, vol. 17, no. 4, 30 April 2012 (2012-04-30) , pages 280-283, XP009514074,
- SU , YU et al.: "Lipid-Lowering and Pleiotropic Effects of Ezetimibe Co-Administration", CHINESE JOURNAL OF NEW DRUGS, vol. 19, no. 8, 31 August 2010 (2010-08-31), pages 666-670, XP9513300,

## Description

The present disclosure relates to the field of biomedicine, more particular to a pharmaceutical composition and use thereof.

### BACKGROUND

Fatty liver, as one of common diffused hepatic diseases, is a metabolic disorder of liver fat in multiple causes, characterized by a condition mainly caused by an excess build-up of fat in liver cells due to increased fat uptake but decreased fat oxidation. The fatty liver has complex causes, generally like alcoholism, obesity, diabetes, or abnormal lipid metabolism, especially in a close relation to hyperlipidemia. With yet not well clarified pathogenesis, fatty liver formation is generally considered to be resulted from imbalance between triglyceride synthesis and low density lipoprotein secretion in the liver cells. The fatty liver usually includes alcoholic fatty liver disease (AFLD) and nonalcoholic fatty liver disease (NAFLD) depending on causes, wherein the NAFLD is a condition where excess fat in form of triglyceride accumulates in the liver cells (i.e. steatosis) with pathogenesis related to insulin resistance (IR) and metabolism syndrome (MS). The NAFLD can progress from nonalcoholic fatty liver (NAFL), to nonalcoholic steatohepatitis (NASH) and then hepatic fibrosis, eventually lead to liver cirrhosis or even liver cancer. NAFLD is currently blooming worldwide. For example, NAFLD has become the most common one among all liver diseases in developed countries like the USA and the Europe, with an estimated 20% to 30% of population with NAFLD which will continue increasing up to 50% by 2030. It is the same in China, where NAFLD has been one of most common chronic liver diseases in rich areas. The morbidity of liver diseases by fatty liver is increasing rapidly in China, where the male between the ages of 40 to 49 years among check-up crowd ranks highest in morbidity (i.e. up to 28%). It becomes more concerned that few patients diagnosed with fatty liver continue reexamination. Thus, the fatty liver has become the second one of all liver diseases (among them viral hepatitis ranks first), with incidence of 10% of total population, where up to 25% of patients with fatty liver may progress to hepatic fibrosis, and approximately 1.5% to 8.0% of the patients further progress to liver cirrhosis even liver cancer. In the case of progression into NASH, the patients with NAFLD will have a greatly increased risk of liver cirrhosis, hepatic failure and liver cancer. It is reported that 20% to 40% of adults have been affected by NAFLD, with around 30% of patients with NAFLD progressed into NASH, where 15% to 25% of patients with NAFLD may progress into liver cirrhosis, and eventually around 30% to 40% of the patients with liver cirrhosis die of liver diseases. In view of the above, fatty liver diseases have become a new challenge in the field of liver disease.

However, no medicament for treating NAFLD has been approved. Currently, drugs for treating NAFLD mainly include insulin sensitizers (for example, biguanides such as Metformin, thiazolidinedione compounds such as Rosiglitazone and Pioglitazone), lipid-lowering agents (such as statins and fibrates), cholagogic drugs (such as ursodeoxycholic acid), liver protection drugs (such as vitamin E, silymarin, acetylcysteine) and traditional Chinese medicine (such as *Fallopia multiflora* (Thunb.) Harald., *Salvia miltiorrhiza Bge., Alisma plantago-aquatica* Linn., *Ligusticum chuanxiong* Hort., *Catsia tora Linn,* or *Crataegus pinnatifida Bunge*). Such various kinds of drugs have single action mechanism and exhibit similar efficacy, with a poor integrated effect, along with side effects and poor tolerance in a patient, which are in need of improvement. At present, there are no known and effective methods for treating NAFLD, and new drugs are still at the stage of clinical trial. Although it has been demonstrated that the insulin sensitizer (such as thiazolidinedione compounds) exhibits efficacy on improvement of liver inflammation and fat content, it is impossible to block the progression of NASH into liver cirrhosis.

RU 2473342 C1 discloses methods for treating non-alcoholic fatty liver disease based on administration of metronidasol, alpha-normix and bifiform. Mudaliar et al., Gastroenterology, 2013, 145, pages 574 to 582 report the efficacy and safety of the farnesoid X receptor agonist obeticholic acid in patients with type 2 diabetes and nonalcoholic fatty acid liver disease. Takeshita et al., Diabetologica, 2014, 57, pages 878 to 890, report the effects of ezetimibe on non-alcoholic fatty liver disease and glucose metabolism based on a randomised controlled trial. Therefore, there is a need to improve the medicament for treating fatty liver diseases.

### SUMMARY

Embodiments of the present disclosure aim to solve at least one of the problems existing in the related art to at least some extent, or to at least provide a useful commercial alternative. For this purpose, the present disclosure provides in embodiments a pharmaceutical composition for effectively treating or preventing nonalcoholic fatty liver disease (NAFLD) and nonalcoholic steatohepatitis (NASH).

In a first aspect, the present invention is directed to a pharmaceutical composition, comprising a first component, being at least one selected from a farnesoid X receptor agonist, which is obeticholic acid, a pharmaceutically acceptable salt and a solvate thereof; and a second component, being at least one selected from a NPC1L1 receptor inhibitor, which is Ezetimibe, a pharmaceutically acceptable salt, and a solvate thereof.

In a preferred embodiment, the pharmaceutical composition comprises the first component in 1 to 100 parts by weight, and the second component in 1 to 100 parts by weight.

In another preferred embodiment, the pharmaceutical composition comprises:
the first component in 2.5 to 50 parts by weight, and the second component in 5 to 50 parts by weight.

In a second aspect, the present invention is directed to a medicament, comprising the pharmaceutical composition according to the first aspect of the present invention as an active ingredient.

In a third aspect, the present invention is directed to the pharmaceutical composition according to the first aspect of the present invention for use in preventing and/or treating nonalcoholic fatty liver disease (NAFLD) or nonalcoholic steatohepatitis (NASH).

In a preferred embodiment, the present invention is directed to the pharmaceutical composition according to the first aspect of the present invention for oral administration, and the first and second components are for administration at the same time or sequentially at different times.

In another preferred embodiment, the present invention is directed to the pharmaceutical composition according to the first aspect of the present invention for administration at a dosage of 1 to 1000 mg per day.

Thus, the pharmaceutical composition provided in embodiments of the present disclosure exhibits efficacy on the reduction in levels of total cholesterol (TC) and/or triglyceride (TG), low density lipoprotein cholesterol (LDL-C), high density lipoprotein cholesterol (HDL-C), and/or alanine aminotransferase (ALT) and/or aspartate aminotransferase (AST) and alkaline phosphatase (ALP), and liver index, further has significant effect on the prevention and/or treatment of NAFLD. Particularly, the pharmaceutical composition exhibits excellent effect on the prevention and/or treatment of nonalcoholic fatty liver disease and nonalcoholic steatohepatitis.

The farnesoid X receptor agonist is obeticholic acid (OCA). The NPC1L1 receptor inhibitor is Ezetimibe. The pharmaceutical composition including obeticholic acid in combination with Ezetimibe is capable of further improving effect on the prevention and/or treatment of nonalcoholic fatty liver disease and nonalcoholic steatohepatitis.

Ezetimibe is a selective cholesterol absorption inhibitor, which functions on brush border of intestinal mucosa via specifically binding to NPC1L1 transport protein on the intestinal mucosa, thus selectively inhibiting intake of exogenous cholesterol and related phytosterols in small intestine, thereby reducing storage of cholesterol in liver, levels of total cholesterol and low density lipoprotein cholesterol in plasma, as well increasing level of high density lipoprotein cholesterol in plasma. Further, Ezetimibe exhibits poor effect on intake inhibition of other nutrients, such as vitamins, fats, proteins, or sugars , thus mainly aiming at hyperlipidemia caused by diet. Furthermore, Ezetimibe has no effect on inhibition of endogenous cholesterol in liver, unlike statins. According to current technical development, administration in combination with statins is still the focus study for Ezetimibe, which is useful in reducing the level of low density lipoprotein cholesterol in patients with hypercholesterolemia caused by diabetes or metabolic syndromes, as well decreasing lipids to prevent cardiovascular events caused by atherosclerosis.

It has been shown by recent investigations that in addition to intestinal villi crypt, the target site NPC1L1 receptor protein of Ezetimibe is also expressed and distributed in liver of primates and human beings, suggesting that Ezetimibe is capable of functioning on NPC1L1 receptor protein in the liver, thus facilitating removal of low density lipoprotein (LDL), chylomicron containing great amounts of lipids, or very low density lipoprotein (VLDL) from liver or gall, as well improving alleviation of fatty liver. Despite of proposal on utilization of Ezetimibe as a promising candidate for the treatment of NAFLD due to its excellent performance on reduction in LDL-C, Ezetimibe has not yet been demonstrated to be a therapeutic drug for NAFLD.

In specific embodiments of the present disclosure, Ezetimibe which is selected for the pharmaceutical composition of the present disclosure is of high grade of safety. Ezetimibe can be absorbed rapidly after oral administration, followed by saccharification into a more active product in bowel and liver, which acts on the NPC1L1 receptor protein repeatedly via hepatoenteral circulation. It is observed by drug metabolism experiment of Ezetimibe with ¹⁴C as a marker that Ezetimibe is barely metabolized by a primary drug metabolic enzyme (i.e. cytochrome p450), thus hardly interacting with other drugs.

It is discovered by present inventors through massive investigations that the combined administration of Ezetimibe and obeticholic acid is able to significantly strengthen the effect of obeticholic acid on alleviation of nonalcoholic fatty liver disease and nonalcoholic steatohepatitis, as well to reduce side effects (such as, LDL increase) of administering obeticholic acid alone. Accordingly, with the combined administration of Ezetimibe and obeticholic acid, the deficiencies of obeticholic acid in the treatment of nonalcoholic fatty liver disease and nonalcoholic steatohepatitis are well compensated. Furthermore, Ezetimibe and obeticholic acid when administered in combination do not interact with each other. Therefore, the pharmaceutical composition is in great safety.

In specific embodiments of the present disclosure, the pharmaceutical composition described above including obeticholic acid and Ezetimibe is found by the present inventors based on the following findings.

Firstly, obeticholic acid is a semisynthetic chenodeoxycholic acid, which is largely expressed in cells participated in bilirubin metabolism in liver, intestine, or kidney, with bile acid as its natural ligand. Compared to a placebo control, patients in an obeticholic acid treatment group have higher levels of total cholesterol and low density lipoprotein, and lower level of high density lipoprotein, some of who have to receive lipid intervention or even to stop administration in advance. The biggest concern about administration of obeticholic acid is long-term safety, due to increase in cholesterol and insulin resistance or risk of arteriosclerosis after administration, thus resulting in cardiovascular events, therefore obeticholic acid is still in a need of further research for treatment of diseases.

In order to reduce the side effects described above, it is usually conceived by skilled in the art to administrate obeticholic acid in combination with statins. According to current technical development, the statins, as one of most widely used lipid lowering drugs have been studied extensively for administration alone. At present, patients with cardiovascular diseases mainly benefit from statins on its cholesterol lowering effect. Further, with massive investigations *in vivo* and *in vitro,* it is demonstrated that statins also exhibit anti-inflammatory effect independent of lipid lowering effect, characterized by the regulation of immunity, anti-oxidation, anti-thrombosis, or antibacterial effect. Because of the close relationship between inflammation and redox mechanisms, and pathogenesis of NAFLD and NASH, the statins are recommended for the treatment of NAFLD and NASH with dyslipidemia by Guidelines in many countries.

At present, the efficacy and safety of statins for the treatment of NAFLD and NASH have been studied well. Statins when used for treatment reduce transaminase and hepatic steatosis, but have no efficacy on amelioration of liver fibrosis, suggesting that the statins are capable of delaying progression from NAFLD to NASH. In other words, statins exhibit certain efficacy on treatment of NAFLD and NASH with dyslipidemia (i.e. high LDL-C diseases). However, the above conclusions come from clinical trials in small sample volume with insufficient time period for follow-ups and absence of histological examination. On one hand, statins have severe safety risks, such as muscular disorders, which may be considered to be the most severe side effect. The muscular disorders have incidence and severity in a positive correlation with dose of statins, and are mainly characterized by myalgia or muscle weakness, associated with increased creatine kinase (CK) level which may be up to more than 10 times of the upper limit over the normal level, also along with symptoms like fever or general malaise. If the muscular disorders are not detected in time and the administration of statins continues, it is possible to result in rhabdomyolysis and acute renal failure. Besides, statins may cause abnormal blood glucose, liver enzyme abnormality, and memory and cognitive disorders. On the other hand and it is the most critical that, statins reduce the intracellular cholesterol synthesis by blocking mevalonate pathway in cells via competitively inhibiting endogenous cholesterol synthesis rate-limiting enzyme (HMG-CoA reductase), thus stimulating low-density lipoprotein receptors on the membrane surface of cells (i.e. mainly liver cells) in a feedback way so as to increase the number and activity of the low-density lipoprotein receptors, thereby resulting in an increase in clearance of serum cholesterol and a decrease in serum cholesterol level. However, statins do not function well on exogenous cholesterol, whereas blood cholesterol of patients with fatty liver largely derives from exogenous cholesterol due to dietary intake. Furthermore, adverse reactions often occur when statins are administered in combination with other drugs, such as fibrates, niacin, cyclosporine, or human immunodeficiency virus (HIV) protease inhibitors. In view of the above, it is believed by the present inventors that the general combination of obeticholic acid with statins is not a suitable choice.

For the purpose above, it is surprisingly found by the present inventors through lots of experiments that farnesoid X receptor agonists and cholesterol absorption inhibitors have synergistic effects, particularly the combined administration of obeticholic acid and Ezetimibe exhibits unexpected effect on the treatment of NAFLD and NASH, more particularly the composition of obeticholic acid and Ezetimibe is capable of reducing elevated LDL, a side effect of administration of obeticholic acid alone. Thus, it is expected to develop the composition for use in treatment of nonalcoholic fatty liver disease and nonalcoholic steatohepatitis.

In specific embodiments of the present disclosure, the pharmaceutical composition described in above embodiments includes 1 to 100 parts by weight of the first component, and 1 to 100 parts by weight of the second component, thus being capable of further improving the synergistic effect and thereby improving the effect on the treatment of NAFLD and NASH under such the weight ratio.

In specific embodiments of the present disclosure, the pharmaceutical composition described in above embodiments preferably includes 2.5 to 50 parts by weight of the first component, and 5 to 50 parts by weight of the second component, thus being capable of further improving the synergistic effect and thereby improving the effect on the treatment of NAFLD and NASH under such the weight ratio.

In another aspect, the present disclosure in embodiments provides a medicament, including the pharmaceutical composition as described above as an active ingredient. The medicament exhibits pharmacological efficacy of the active ingredient, thus further possessing all the efficacy of the pharmaceutical composition. Specifically, the medicament exhibits effect on the reduction in levels of total cholesterol and/or triglyceride, low density lipoprotein cholesterol, high density lipoprotein cholesterol, and/or alanine aminotransferase and/or aspartate aminotransferase and alkaline phosphatase, and liver index, further has significant effect on the prevention and/or treatment of NAFLD. Particularly, the medicament exhibits excellent effect on the prevention and/or treatment of nonalcoholic fatty liver disease and nonalcoholic steatohepatitis.

In specific embodiments of the present disclosure, the medicament in addition to the active ingredient as described above, further includes a pharmaceutically acceptable carrier. According to embodiments of the present disclosure, the pharmaceutically acceptable carrier is at least one selected from the group consisting of pharmaceutically acceptable solvents, propellants, solubilizers, cosolvents, emulsifiers, colorants, binders, disintegrators, fillers, lubricants, wetting agents, osmotic pressure regulators, stabilizers, flow aids, flavoring agents, preservatives, suspending agents, coating materials, fragrances, anti-adhesives, integrators, penetration enhancers, pH value modifier, buffer, plasticizers, surfactants, foaming agents, antifoaming agents, thickeners, inclusion agents, moisturizers, absorbents, diluents, flocculants and deflocculants, filter aids, release retarders, polymer framework materials and film-forming materials, thus enabling the pharmaceutical composition to be formulated into a dosage form suitable for administration in clinic.

In specific embodiments of the present disclosure, the medicament as described above can be in any dosage form suitable for oral administration. The medicament provided in embodiments of the present disclosure including the pharmaceutical composition as an active ingredient, has a dosage form for oral administration including tablets, sublingual tablets, effervescent tablets, coated tablets, sugar-coated tablets, dispersible tablets, enteric coated tablets, granules, gelatin capsules, enteric capsules, sustained release capsules, controlled release capsules and oral liquids, preferably, tablets or capsules.

In specific embodiments of the present disclosure, in the pharmaceutical composition as the active ingredient of the medicament described above, the first component is of an amount of 1 mg to 100 mg, preferably 2.5 mg to 50 mg, and the second component is of an amount of 1 mg to 100 mg, preferably 5 mg to 50 mg.

In a specific embodiment of the present disclosure, in the pharmaceutical composition as the active ingredient of the medicament described above, the first component is of an amount of 1 mg, and the second component is of an amount of 1 mg.

In a further aspect, the present disclosure in embodiments provides the pharmaceutical composition as described above for preventing and/or treating nonalcoholic fatty liver disease (NAFLD).

In a further aspect, the present disclosure in embodiments provides the pharmaceutical composition as described above for preventing and/or treating nonalcoholic steatohepatitis (NASH).

In specific embodiments of the present disclosure, the pharmaceutical composition is for oral administration, and the first and second components are for concomitant administration at the same time or sequentially at different times.

In specific embodiments of the present disclosure, the pharmaceutical composition is for administration in a dosage of 1 to 1000 mg per day.

The additional aspects and advantages of the present disclosure will be given partly from the following description, part of which will become apparent from the description or understood from the practice of the present disclosure.

### BRIEF DESCRIPTION OF THE DRAWINGS

The drawings aim to provide further understanding of the present disclosure and constitute a part of the description, which are intended to explain the present disclosure in combination with specific embodiments described below and not to limit the scope of the present disclosure. In which:
Figure 1 is graphs showing effect on morphology of liver tissues of NAFLD-modeled golden hamsters after 4 weeks of administration according to example 4 of the present disclosure.
Figure 2 is graphs showing effect on liver tissue color of NAFLD-modeled golden hamsters after 4 weeks of administration according to example 4 of the present disclosure.
Figure 3 is graphs showing pathological sections of liver tissues of NASH-modeled golden hamsters after administration obeticholic acid in combination with Ezetimibe.
Figure 4 is a graph showing effect of obeticholic acid in combination with Ezetimibe on HBVcccDNA reduction in NASH-modeled golden hamsters.

### DETAILED DESCRIPTION

The embodiments of the present disclosure are described in detail below. Such embodiments are explanatory, and aim at to explain the present disclosure rather than to be construed to limit the present disclosure. If the specific technology or conditions are not specified in the examples, a step will be performed in accordance with the techniques or conditions described in the literature in the art or in accordance with the product instructions. If the manufacturers of reagents or instruments are not specified, the reagents or instruments may be commercially available.

### Example 1 Preparation of obeticholic acid

1.3 kg of 3α-hydroxy-6-ethylidene-7-keto-5β-cholanoic acid, 13.0 kg of purified water, 0.65 kg of 50% aqueous sodium hydroxide solution and 0.13 kg of 10% palladium on carbon were added into a reaction vessel. The reaction mixture was hydrogenated at 1 to 3 atmospheres until the reaction was completed. After gas replacement with nitrogen, the reaction solution was filtered to recover the catalyst, the filtrate was collected and transferred to the reaction vessel where it was further stirred under an increased temperature of 95 to 100°C, until the reaction was completed. 11.5 kg of butyl acetate was added, and about 5.2 kg of aqueous citric acid solution was used to adjust the pH value to 4 to 5 with stirring. After stirring for another 0.5 hours, the mixture was allowed to stand for separation. The organic phase was separated, washed with water, followed by addition of activated carbon and treatment at around 55°C for about 0.5 hours. Subsequently, the activated carbon was filtered off, and the filtrate was concentrated under reduced pressure to precipitate a large amount of solids, which were further filtered. The filter cake obtained was washed with 0.6 kg of butyl acetate, and dried under vacuum at a temperature not higher than 60°C, thus obtaining 1.2 kg of 3α-hydroxy-6α-ethyl-7-keto-5β-cholanoic acid.

To the reaction vessel, 1.15 kg of 3α-hydroxy-6α-ethyl-7-keto-5β-cholanoic acid, 9.2 kg of purified water and 1.1 kg of 50% aqueous sodium hydroxide solution were added. The mixture was heated to around 80°C and stirred until all the materials were dissolved. Afterwards, sodium borohydride (0.104 kg) dissolved in 5% aqueous sodium hydroxide was added to the reaction vessel. The mixture was still stirred under an increased temperature of 95 to 105°C, until the reaction was completed. After the mixture was cooled to around 55°C, 8.1 kg of butyl acetate was added and 8.6 kg of 50% aqueous citric acid solution was used to adjust the pH value to 4 to 5 with stirring. Then the mixture was allowed to stand for separation. The organic phase was separated, washed with water, and concentrated under reduced pressure. The solid collected by filtration was recrystallized with butyl acetate, and dried under vacuum to give 0.75 kg of crude product, i.e. crude obeticholic acid. Thereafter, the crude product was added to 6.0 kg of purified water and 0.17 kg of 50% aqueous sodium hydroxide solution, with stirring for dissolution. After filtration through a filter membrane, the filtrate was added to a diluted hydrochloric acid system (formulated by 16.7 kg of purified water and 0.25 kg of 37% concentrated hydrochloric acid), with a system temperature above 40°C. After stirring for another 1 hour at a temperature between 40°C and 45°C, the mixture was cooled down to around 20°C, followed by filtration to give a filter cake, which was washed with purified water until the pH value reached 6.0 to 7.0 and dried under vacuum at a temperature around 50°C, thus obtaining 0.7 kg of obeticholic acid.

### Example 2 Preparation of Ezetimibe

(4S)-3-[(5S)-5-(4-fluorophenyl)-5-hydroxy-1-oxopentyl]-4-phenyl-2-oxazolidinone (2.0 kg), 4-[[(4-fluorophenyl)imine]methyl]-phenol (2.4 kg) and dichloromethane (15.0 kg) were added to a 50 L reaction vessel under nitrogen protection, with a temperature kept below -5°C. N,N-Diisopropylethylamine (DIPEA) (3.8 kg) was added, and Trimethylsilyl chloride (2.3 kg) was added dropwise slowly under stirring. After that, the mixture was kept stirring, until the reaction was completed, thus obtaining a primary raw material called system A.

To a 100 L reaction vessel containing dichloromethane (4.5 kg), titanium tetrachloride (1.26 kg) was added, with a temperature cooled down to around -15°C. After addition of titanium tetraisopropoxide (0.68 kg), the mixture was stirred for 20 minutes and then cooled down to a temperature of -30°C. The TMS protected-primary raw material (i.e. system A) was added dropwise slowly, during which the temperature was controlled not higher than -20°C, followed by stirring vigorously for about 3 hours to complete the reaction.

Isopropanol (16.0 kg) was added dropwise under a controlled temperature below 0°C, followed by addition of dichloromethane (34.5 kg) and stirring for 0.5 hours. After further addition of saturated aqueous tartaric acid solution (35.0 kg), the mixture was warmed naturally to room temperature and stirred for another 3 hours to complete the reaction, with an aqueous phase and an organic phase obtained. After the aqueous phase was extracted with dichloromethane, the combined organic phases were washed with purified water (24.0 kg), followed by addition of 20% aqueous sodium bisulfite solution (35.0 kg) and stirring for about 3 hours, with a first crop of solid precipitated. The first crop of solid was collected by filtration and washed with purified water. The filtrate was transferred into the reaction vessel and an organic phase was collected after separation of the aqueous phase. The organic phase was washed with purified water (16.0 kg), and then concentrated under reduced pressure to remove solvent thoroughly. After that, dichloromethane (5.6 kg) and purified water (4.0 kg) were added, with a second crop of solid precipitated, which was collected by filtration. The first crop of solid and the second crop of solid were combined, and dried, thus obtaining 1.82 kg of (S)-3-((S)-5-(4-fluorophenyl)-5-(trimethylosiloxyl)pentanoyl)-4-phenyl-2-oxazolidinone.

To the 100 L reaction vessel, (S)-3-((S)-5-(4-fluorophenyl)-5-(trimethylsiloxy)pentanoyl)-4-phenyl-2-oxazolidinone (1.82 kg), dichloromethane (10.5 kg) and bis(trimethylsilyl)acetamide (BSA) (2.55 kg) were added, heated to reflux, and stirred until the reaction was completed. After the mixture was cooled down to room temperature, methyl tert-butyl ether (4.9 kg) was added and stirred for 1 hour, followed by addition of tetrabutylammonium fluoride trihydrate (TBAF·3H₂O) (36.4 g) and stirring to complete the reaction. The mixture was concentrated under reduced pressure to remove solvent thoroughly, after which acetone (3.8 kg) was added, and then 2 mol/L of sulfuric acid solution (2.0 kg) was added dropwise at a temperature of 0 to 10°C. The reaction mixture was stirred at room temperature until the reaction was completed. Subsequently, dichloromethane (9.0 kg) and purified water (4.0 kg) were added with stirring, with a third crop of solid precipitated, which was collected by filtration. The filtrate was again transferred into the reaction vessel and an organic phase was collected after separation of the aqueous phase. The organic phase was washed with purified water (10 kg) and stirred at a controlled temperature of 0 to 10°C, so as to give a fourth crop of solid precipitated. The fourth crop of solid was collected by filtration, and combined with the third crop of solid. After dichloromethane (5 kg) and purified water (5 kg) were added into the combined solids, the mixture was subjected to slurrying, followed by cooling down to a temperature of 0 to 5°C and filtration under reduced pressure. The filter cake obtained was dried under vacuum at a temperature of 45 to 55°C. After that, the solid product obtained was dissolved in absolute ethanol (14 kg) and filtered, then the filtrate was concentrated to about half volume under reduced pressure at a temperature of 45 to 55°C. After addition of purified water (6.6 kg), the mixture was cooled down to a temperature of 0 to 5°C for crystallization, and stirred for another 1 hour, followed by filtration under reduced pressure and collection of solids, which were dried under vacuum at a temperature around 50°C, thus obtaining 770 g of Ezetimibe.

### Example 3 Preparation of Ezetimibe-glucuronide

0.091 ml of boron trifluoride diethyl ether was added to a solution containing 3.33 g of 3,4,6-tri-O-acetyl-α-D-glucopyranose 1,2-(methyl orthoacetate) and 4.24 g of 3-O-acetyl ezetimibe in dichloromethane at a temperature of -25°C. After reaction at -20°C for 2 hours, the mixture was warmed to 10°C and kept reacting until the reaction was completed. Subsequently, the mixture was quenched with saturated aqueous ammonium chloride solution, followed by extraction with ethyl acetate. The organic phase was separated, washed with the saturated aqueous ammonium chloride solution, water and aqueous sodium chloride solution, and dried over anhydrous sodium sulfate before further concentrated and purified by silica gel column chromatography, thus obtaining 5.39 g of an intermediate product.

5.08 g of the intermediate was dissolved in 127 ml of methanol and 127 ml of triethylamine, then 445 ml of water was slowly added at room temperature. The resulting clear reaction solution was stirred overnight until the reaction was completed. The mixture was concentrated to remove methanol and triethylamine, followed by addition of 1M hydrochloric acid for acidification and extraction with ethyl acetate. Afterwards, the combined organic phases were washed with 1M hydrochloric acid, water and aqueous sodium chloride solution, and then dried over anhydrous sodium sulfate before further concentrated and purified by silica gel column chromatography and dried again, thus obtaining 2.6 g of Ezetimibe-glucuronide.

### Example 4 Effect of obeticholic acid alone vs. in combination with Ezetimibe on simple high-fat diet-induced NAFLD

### 1. Instruments

Sysmex CHEX-180 automatic biochemical analyzer
Beckman 22R centrifuge
Sartorius CP124S electronic balance
KUDOS SK7200H ultrasound cleaner

### 2. Reagents

The following kits are all commercially available from Shanghai Kehua Bio-Engineering co., Ltd.: TC assay kit (Lot. No. 20150912), TG assay kit (Lot. No. 20151012), LDL-C assay kit (Lot. No. 20151122), HDL-C assay kit (Lot. No. 20150322), ALP assay kit (Lot. No. 20151112) and GLU assay kit (Lot. No. 20150822).

ALT assay kit (Lot. No. ZG5005) or AST assay kit (Lot. No. ZG5004) are commercially available from Sysmex Corporation.

Obeticholic acid and Ezetimibe each are formulated with 10% Kolliphor HS 15 (BASF, Lot. No. 21466288K0) to a desired concentration.

### 3. Animals

78 specific pathogen free (SPF) male golden hamsters were provided, each with a body weight (BW) around 70 to 90 g, and commercially available from Beijing Vital River Laboratory Animal Technology Co., Ltd. Animal permit number is SCXK (Jing) 2012-0001.

### 4. Establishment of the NAFLD-modeled golden hamster

10 golden hamsters were fed with a base diet as a normal group, while 68 golden hamsters were fed with a high-fat diet formulated with 0.3% cholesterol, 12.5% palm oil and 87.2% base diet upon purchased. All of them had free access to water, and kept in a same light rhythm (day time/night time: 10 h/14 h). After 3 weeks of feed, the golden hamsters were fasted for 14 hours, with free access to water, and then sampled for blood in a volume of 0.8 mL from posterior orbital venous plexus the next day. The blood samples added with sodium heparin for anticoagulation were centrifuged at 5000 r·min⁻¹ for 10 minutes, and 300 µL of supernatant for each sample was transferred into an assay cup (in volume of microliter grade) of the Sysmex CHEX-180 automatic biochemical analyzer for determination of levels of TC, TG, LDL-C, HDL-C and ALT, with results for screening of the hamsters.

### 5. Grouping and administration

6 out of 10 golden hamsters fed with the base diet were selected as a normal group, while 30 out of 68 golden hamsters fed with the high-fat diet were grouped into 3 experimental groups randomly (i.e. 10 hamsters for each group), respectively named model group, obeticholic acid group (1 mg/kg) and obeticholic acid (1 mg/kg) + Ezetimibe (1 mg/kg) group. From the first administration at PM 4:00 of the day after grouping, the 3 experimental groups were started to be each administered in a dosage of 10 mL·kg⁻¹, where the model group was administered with 10% Kolliphor in an equal volume. During administration, the normal group was still fed with the base diet, and the 3 experimental groups were fed with the high-fat diet. At 2 weeks of administration, all the golden hamsters were fasted but accessible to water overnight, and anesthetized with ethyl ether on the morning of the next day for collection of blood samples (in a volume of 0.8 mL) from the posterior orbital venous plexus. The blood samples added with sodium heparin for anticoagulation were centrifuged at 5000 r·min⁻¹ for 10 minutes, and 300 µL of plasma for each sample was transferred into the Sysmex CHEX-180 automatic biochemical analyzer for determination of levels of TC, TG, LDL-C, HDL-C, ALT, AST and ALP. At 4 weeks of administration, plasma of each hamster was collected for determination of levels of TC, TG, LDL-C, HDL-C, ALT, AST and ALP. Afterwards, the hamsters were anesthetized with ethyl ether deeply, followed by weighting and laparotomy by an abdominal median incision for observation of size, color and texture of liver. After photographed, the liver was taken out, washed with saline and dried with filter paper, and weighted for calculation of liver index. Subsequently, left lobe of liver was cut out quickly and placed into 10% of neutral buffered formalin for immobilization, followed by preparation of paraffin sections (with HE staining) for observation of change of liver tissue pathology.

### 6. Statistical analyses

The results are represented in *X̅*±s, and comparison among groups is performed by t-test.

### 7. Results

Table 1 shows results of levels of TC, TG, LDL-C, HDL-C, ALT, AST and ALP in plasma of individual golden hamsters in each group of after 2 weeks of administration. Compared with the normal group, the levels of TC, TG, LDL-C, HDL-C, ALT, AST and ALP in plasma are significantly higher in the model group (*P*<0.05 or *P*<0.01). Compared with the model group, the obeticholic acid group shows that the level of AST in golden hamster plasma is significantly reduced (*P*<0.05), but the levels of TC, TG, LDL-C, HDL-C, ALT and ALP are not changed obviously (*P*>0.05); while the obeticholic acid + Ezetimibe group shows that the levels of TC, TG, LDL-C, HDL-C, ALT and AST are significantly reduced (*P*<0.05 or *P*<0.01), but the level of ALP in plasma is not changed obviously (*P*>0.05).

Table 2 shows results of levels of TC, TG, LDL-C, HDL-C, ALT, AST and ALP in plasma as well as liver index of individual golden hamsters in each group after 4 weeks of administration. Compared with the normal group, the levels of TC, TG, LDL-C, HDL-C, ALT and AST in plasma as well as the liver index are significantly higher in the model group (*P*<0.01), but the ALP level is not varied significantly (*P*>0.05). Compared with the model group, the obeticholic acid group shows that the levels of TC, LDL-C, ALT and AST in hamster plasma are significantly reduced (*P*<0.05 or *P*<0.01) although still in relative high values, but the levels of TG, HDL-C and ALP as well as the liver index are not changed obviously (*P*>0.05); while the obeticholic acid + Ezetimibe group shows that the liver index (*P*<0.01), and the levels of TC, TG, LDL-C, HDL-C, ALT, AST and ALP are significantly reduced (*P*<0.01).

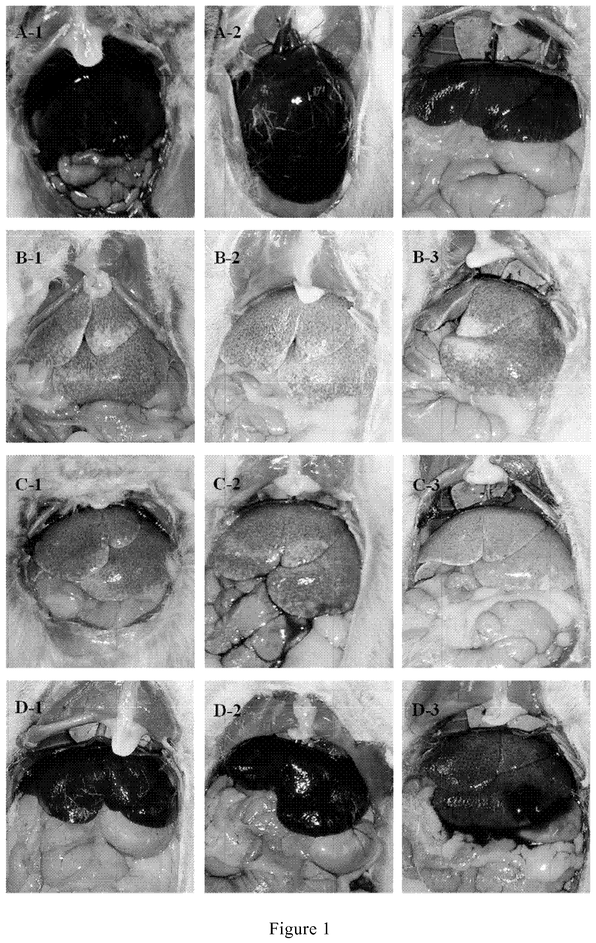

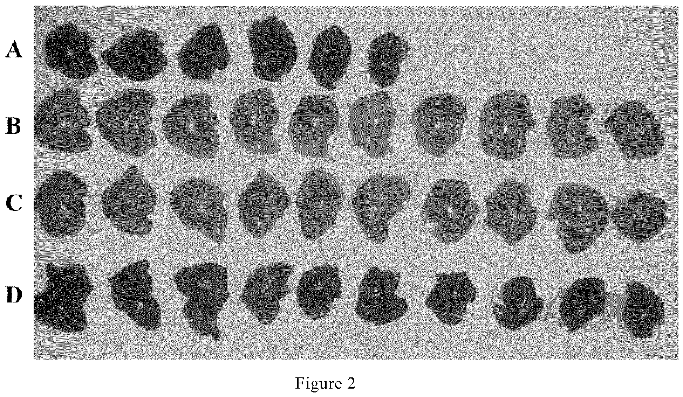

Liver tissue samples from individual golden hamsters in each group after 4 weeks of administration are shown in Figure 1 and Figure 2. Figure 1 shows effect on morphology of liver tissues of the NAFLD-modeled golden hamsters after 4 weeks of administration, where A-1,2,3 represents the normal group, B-1,2,3 represents the model group, C-1,2,3 represents the obeticholic acid group and D-1,2,3 represents the obeticholic acid + Ezetimibe group; and 1,2,3 are from random golden hamsters in each corresponding group. Figure 2 shows effect on liver tissue color of the NAFLD-modeled golden hamsters after 4 weeks of administration, where A represents the normal group, B represents the model group, C represents the obeticholic acid group and D represents the obeticholic acid + Ezetimibe group. According to general observation, it can be seen that the liver tissues in the normal group are bright red, with sharp edges, smooth surfaces and soft textures; whereas in contrast the liver tissues in the model group are rough, reddish yellow or creamy yellow, even tending to whiten, also have greatly bigger and full shapes and tense outer membranes, with dull edges, brittle textures and greasy touch. The liver tissues seem not obvious different in morphology between the obeticholic acid group and the model group; while the liver tissues in the obeticholic acid + Ezetimibe group are rosier and more elastic than the model group, and the liver appearance is close to the normal group.

The results indicate that the combined administration of obeticholic acid and Ezetimibe has unexpected effect on the treatment of the NAFLD-modeled golden hamsters, but the administration of obeticholic acid alone does not have obvious therapeutic effect.

**Table 1 Effects on blood lipid level and liver function index of the NAFLD-modeled golden hamsters after 2 weeks of administration (x̅±s)**

| Groups | *n* | TC /mmol·L⁻¹ | TG /mmol· L⁻¹ | LDL-C /mmol L⁻¹ | HDL-C /mmol· L⁻¹ | ALT /U·L⁻¹ | AST /U·L⁻¹ | ALP /U·L⁻¹ |
|---|---|---|---|---|---|---|---|---|
| Normal group | 7 | 3.05±0.89 | 1.03±0.43 | 1.69±0.60 | 1.45±0.38 | 68.57±19.78 | 60.14±8.88 | 164.00±21.20 |
| Model group | 10 | 16.51±4.57^{##} | 1.95±0.69^{##} | 9.28±7.20^{#} | 2.87±0.27^{##} | 158.33±44.81^{##} | 75.67±11.67^{#} | 202.44±14.54^{##} |
| Obeticholic acid group | 10 | 14.32±3.15 | 2.20±0.52 | 7.07±3.22 | 2.70±0.37 | 120.50±41.78 | 59.20±14.01* | 190.20±26.30 |
| Obeticholic acid + Ezetimibe group | 10 | 5.87±0.88** | 1.34±0.42* | 2.57±0.45** | 2.29±0.25** | 74.50±14.58** | 58.40±19.11* | 193.00±22.17 |

| | | | | | | | | |
|---|---|---|---|---|---|---|---|---|
| Noted: in comparison of the normal group, ^{#}*P*<0.05 and ^{##}*P*<0.01; in comparison of the model group, **P*<0.05 and *^{**}P*<0.01. | | | | | | | | |

**Table 2 Effects of blood lipid level, liver function indexes and liver index of the NAFLD-modeled golden hamsters after 4 weeks of administration (x̅±s)**

| Groups | *n* | TC /mmol· L⁻¹ | TG /mmol· L⁻¹ | LDL-C /mmol· L⁻¹ | HDL-C /mmol· L⁻¹ | ALT /U·L⁻¹ | AST /U·L⁻¹ | ALP /U·L⁻¹ | liver index (%) |
|---|---|---|---|---|---|---|---|---|---|
| Normal group | 6 | 3.71±0.29 | 0.76±0.24 | 1.85±0.51 | 1.96±0.15 | 80.17±13.04 | 62.33±7.58 | 185.50±29.70 | 3.43±0.56 |
| Model group | 10 | 29.55±4.62 ## | 2.67±1.38 ## | 30.50±11.19 ## | 3.33±0.58 ## | 220.40±55.42 ## | 110.50±27.61 ## | 190.30±19.82 | 5.35±0.18 ## |
| Obeticholic acid group | 10 | 22.89±5.91 * | 2.07±0.97 | 16.04±13.52 * | 3.51±0.71 | 165.20±44.90 * | 80.80±13.13 ** | 196.30±18.91 | 5.15±0.33 |
| Obeticholic acid + Ezetimibe group | 10 | 3.34±0.35 ** | 0.92±0.20 ** | 0.96±0.37 ** | 2.04±0.29 ** | 86.70±65.11** | 55.50±17.07 ** | 147.00±27.53 ** | 3.22±0.39 ** |

| | | | | | | | | | |
|---|---|---|---|---|---|---|---|---|---|
| Noted: in comparison of the normal group, *^{#}P*<0.05 and ^{##}*P*<0.01; in comparison of the model group, **P*<0.05 and ***P*<0.01. | | | | | | | | | |

In view of the above, the combined administration of the farnesoid X receptor agonist obeticholic acid and the cholesterol absorption inhibitor Ezetimibe exhibits unexpected effect on the treatment of NAFLD and NASH, especially the pharmaceutical composition including obeticholic acid and Ezetimibe is capable of reducing side effects (such as, LDL increase) of administration of obeticholic acid alone, thus it is expected to develop the pharmaceutical composition for use in treatment of nonalcoholic fatty liver disease, nonalcoholic steatohepatitis and hyperlipidemia fatty liver. Further, there have not yet existed reports on treatment with a pharmaceutical composition related to obeticholic acid, let alone the combination of obeticholic acid and Ezetimibe. According to embodiments of the present disclosure, it is discovered by the present inventors through animal models that obeticholic acid and Ezetimibe when administrated in combination give a synergistic effect, and do not interact with each other, thus being in great safety. Therefore, the combined administration of obeticholic acid and Ezetimibe definitely contributes to the treatment of diseases such as nonalcoholic fatty liver disease, nonalcoholic steatohepatitis and hyperlipidemia fatty liver.

### Example 5 Effect of obeticholic acid in combination with Ezetimibe vs. obeticholic acid alone and Ezetimibe alone on NASH

The experimental method is referred to the Example 4, except for addition of Ezetimibe group (0.3 mg/kg), dosage change to 5 mg/kg obeticholic acid for the obeticholic acid group, as well as dosage change to 5 mg/kg obeticholic acid and 0.3 mg/kg Ezetimibe for the obeticholic acid + Ezetimibe group. Specifically, a normal group (A), a model group (B), an obeticholic acid group (C), an obeticholic acid + Ezetimibe group (D) and an Ezetimibe group (E) are included in this example.

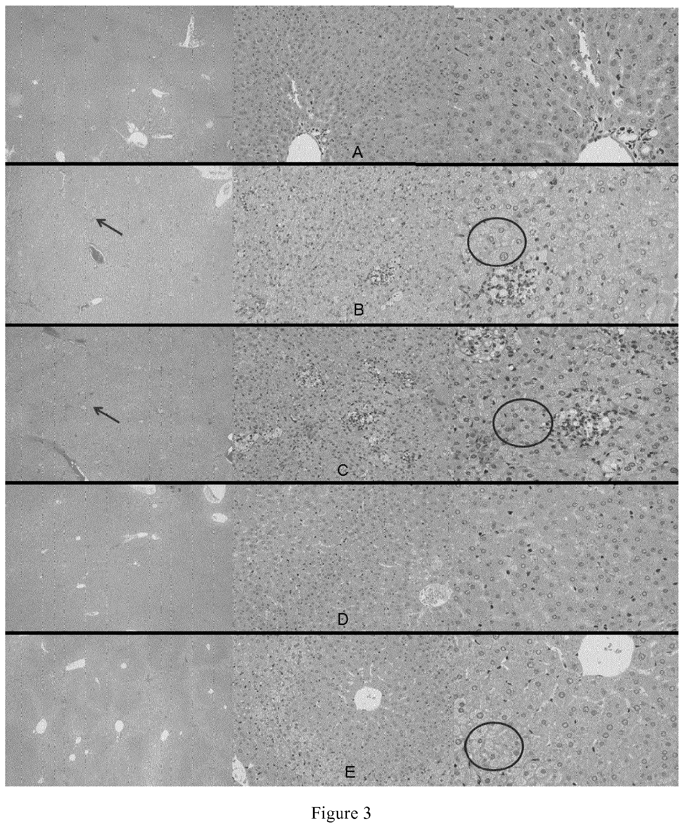

Liver tissue samples from individual golden hamsters after treatment were subjected to paraffin-sectioning, with results shown in Figure 3.

The findings are demonstrated by the NASH-molded golden hamsters as follows:
1) Obeticholic acid alone slightly reduces levels of liver function enzymes (i.e. alanine aminotransferase and aspartate aminotransferase in this context) in plasma, but slightly increases levels of triglyceride, cholesterol and LDL. Further, it can be seen from pathological sections of liver tissues that obeticholic acid alone is capable of reducing fat content in liver cells significantly, with greatly decreased fat bands of liver, but has no effect on the decrease in infiltration and aggregation of inflammatory cells, nor on blocking the progression of liver fibrosis.
2) Ezetimibe alone reduces levels of triglyceride, cholesterol, LDL, liver function enzymes in plasma significantly, but just reduces ALP level slightly. Further, it can be seen from pathological sections of liver tissues that Ezetimibe alone is capable of ameliorating the infiltration and aggregation of inflammatory cells, but not reducing fat content in liver significantly nor removing fat from the liver.
3) The combined administration of obeticholic acid and Ezetimibe reduces levels of triglyceride, cholesterol and LDL in plasma significantly, as well reduces ALP level slightly. Further, it can be seen from pathological sections of liver tissues that such the combined administration is capable of reducing fat content in liver cells significantly, with greatly decreased fat bands, as well reducing infiltration and aggregation of inflammatory cells significantly. Thus, it is speculated that the combined administration of obeticholic acid and Ezetimibe is capable of decreasing cardiovascular risk caused by increases in cholesterol and LDL caused by administration of obeticholic acid alone to patients with NASH in clinic, also significantly strengthening effects on decreases in liver function enzymes as well as infiltration of inflammatory cells, which cannot be provided by administration of obeticholic acid alone, thus slowing down the progression of liver fibrosis.

### Example 6 Effect of obeticholic acid in combination with Ezetimibe vs. obeticholic acid alone and Ezetimibe alone on copy numbers of HBV DNA

### 1. Materials

HepG2, a hepatocellular carcinoma cell, is provided by China Center for Type Culture Collection in Wuhan (Preservation No: GDC024).

Fetal bovine serum (FBS) is commercially available from Gibco Company, USA.

Medium DMEM is commercially available from Gibco Company, USA.

Lipofectamine™2000 transfection reagent is commercially available from Life Technologies, USA.

PCR-Fluorescence Quantification Kit for Hepatitis B Virus (HBV) is commercially available from Shanghai Kehua Bio-Engineering co., Ltd.

### 2. Cell culture condition

The HepG2 cells were cultured with the medium DMEM containing 10% of FBS at a temperature of 37°C under 5% (in volume) of CO₂ and saturated humidity.

### 3. Grouping

For a negative control group, HepG2 cells, dimethyl sulfoxide (DMSO) for replacement of drugs in an equal volume, and the medium DMEM containing 10% of FBS in an equal volume were used.

For an Obeticholic acid group, where the obeticholic acid is administered alone, HepG2 cells, an obeticholic acid solution in final concentrations of 2 µmol/L or 10 µmol/L (formulated with DMSO and the medium DMEM containing 10% of FBS) in an equal volume were used.

For an obeticholic acid + Ezetimibe group, HepG2 cells, a mixture of obeticholic acid and Ezetimibe formulated with DMSO and the medium DMEM containing 10% of FBS (in final concentrations including 2 µmol/L obeticholic acid + 2µmol/L Ezetimibe, 2 µmol/L obeticholic acid + 10 µmol/L Ezetimibe, and 10 µmol/L obeticholic acid + 2 µmol/L Ezetimibe) in an equal volume were used.

### 4. Methods

The HepG2 cells in logarithmic growth phase were seeded into a 24-well plate at a density of 4×10⁵ cells/well and in a volume of 0.5 ml per well, then cultured at 37°C under 5%CO₂ and saturated humidity for 6 hours, with cells having good adhesion and growth. Thereafter, the medium in individual wells of the plate was removed and fresh medium was supplemented, followed by addition of a mixture containing plasmid DNA with complete HBV genome (i.e. pBlue-HBV1.3), where the mixture was prepared by mixing 0.8 µg of the plasmid, 2 µL of the Lipo2000 transfection reagent and 100 µL of Opti-MEM® Reduced-Serum Medium, and standing for 20 minutes. After culture for 12 hours, the medium in each well was discarded, and the formulated solution with or without drug(s) for individual group as described above was added into corresponding wells, with each well in triplicate. The mixture was cultured at 37°C under 5%CO₂ and saturated humidity for another 48 hours, followed by collection of cells for extraction of HBV DNA (i.e. Replicative intermediate, closed circular DNA, in short cccDNA) and further for quantitative PCR detection.

### 5. Results

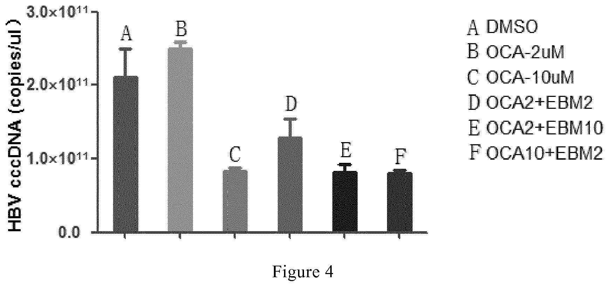

It is demonstrated by Figure 4 that the combined administration of obeticholic acid and Ezetimibe reduces the copy numbers of HBV cccDNA significantly, suggesting that the pharmaceutical composition may also exhibit good effect on the treatment of patients with NASH caused by HBV. In the Figure 4, DMSO stands for dimethyl sulfoxide, OCA stands for obeticholic acid and the EBM stands for Ezetimibe.

## Claims

1. A pharmaceutical composition, comprising
a first component, being at least one selected from a farnesoid X receptor agonist, which is obeticholic acid, a pharmaceutically acceptable salt and a solvate thereof; and
a second component, being at least one selected from a NPC1L1 receptor inhibitor, which is Ezetimibe, a pharmaceutically acceptable salt, and a solvate thereof.

2. The pharmaceutical composition according to claim 1, wherein the pharmaceutical composition comprises:
the first component in 1 to 100 parts by weight, and
the second component in 1 to 100 parts by weight.

3. The pharmaceutical composition according to claim 1, wherein the pharmaceutical composition comprises:
the first component in 2.5 to 50 parts by weight, and
the second component in 5 to 50 parts by weight.

4. A medicament, comprising the pharmaceutical composition as defined in any one of claims 1 to 3 as an active ingredient.

5. The pharmaceutical composition as defined in any one of claims 1 to 3 for use in preventing and/or treating nonalcoholic fatty liver disease (NAFLD).

6. The pharmaceutical composition as defined in any one of claims 1 to 3 for use in preventing and/or treating nonalcoholic steatohepatitis (NASH).

7. The pharmaceutical composition for use according to claim 5 or 6, wherein the pharmaceutical composition is for oral administration, and the first and second components are for administration at the same time or sequentially at different times.

8. The pharmaceutical composition for use according to any one of claims 5 to 7, wherein the pharmaceutical composition is for administration at a dosage of 1 to 1000 mg per day.

## Patentansprüche

1. Pharmazeutische Zusammensetzung, umfassend
eine erste Komponente, die mindestens eine ist, ausgewählt aus einem Farnesoid-X-Rezeptor-Agonisten, der Obeticholsäure, ein pharmazeutisch akzeptables Salz und ein Solvat davon ist; und
eine zweite Komponente, die mindestens eine ist, ausgewählt aus einem NPC1L1-Rezeptor-Inhibitor, der Ezetimibe, ein pharmazeutisch akzeptables Salz und ein Solvat davon ist.

2. Pharmazeutische Zusammensetzung nach Anspruch 1, wobei die pharmazeutische Zusammensetzung umfasst:
die erste Komponente in 1 bis 100 Gewichtsteilen und
die zweite Komponente in 1 bis 100 Gewichtsteilen.

3. Pharmazeutische Zusammensetzung nach Anspruch 1, wobei die pharmazeutische Zusammensetzung umfasst:
die erste Komponente in 2,5 bis 50 Gewichtsteilen und
die zweite Komponente in 5 bis 50 Gewichtsteilen.

4. Arzneimittel, umfassend die pharmazeutische Zusammensetzung wie in einem der Ansprüche 1 bis 3 definiert als Wirkstoff.

5. Pharmazeutische Zusammensetzung, wie in einem der Ansprüche 1 bis 3 definiert, zur Verwendung bei der Vorbeugung und/oder Behandlung von nichtalkoholischer Fettlebererkrankung (NAFLD).

6. Pharmazeutische Zusammensetzung, wie in einem der Ansprüche 1 bis 3 definiert, zur Verwendung bei der Vorbeugung und/oder Behandlung von nichtalkoholischer Steatohepatitis (NASH).

7. Pharmazeutische Zusammensetzung zur Verwendung nach Anspruch 5 oder 6, wobei die pharmazeutische Zusammensetzung zur oralen Verabreichung bestimmt ist und die erste und die zweite Komponente zur Verabreichung zur gleichen Zeit oder nacheinander zu unterschiedlichen Zeiten bestimmt sind.

8. Pharmazeutische Zusammensetzung zur Verwendung nach einem der Ansprüche 5 bis 7, wobei die pharmazeutische Zusammensetzung zur Verabreichung in einer Dosierung von 1 bis 1000 mg pro Tag bestimmt ist.

## Revendications

1. Composition pharmaceutique comprenant
un premier composant, qui est au moins l'un choisi parmi un agoniste de récepteur farnésoïde X, lequel est l'acide obéticholique, un sel pharmaceutiquement acceptable et un solvate de celui-ci ; et
un deuxième composant, qui est au moins l'un choisi parmi un inhibiteur de récepteur NPC1L1, lequel est l'ézétimibe, un sel pharmaceutiquement acceptable et un solvate de celui-ci.

2. Composition pharmaceutique selon la revendication 1, dans laquelle la composition pharmaceutique comprend :
le premier composant à raison de 1 à 100 parties en poids, et
le deuxième composant à raison de 1 à 100 parties en poids.

3. Composition pharmaceutique selon la revendication 1, dans laquelle la composition pharmaceutique comprend :
le premier composant à raison de 2,5 à 50 parties en poids, et
le deuxième composant à raison de 5 à 50 parties en poids.

4. Médicament comprenant, en tant que principe actif, la composition pharmaceutique telle que définie dans l'une quelconque des revendications 1 à 3.

5. Composition pharmaceutique selon l'une quelconque des revendications 1 à 3, pour une utilisation dans la prévention et/ou le traitement d'une stéatopathie non alcoolique (NAFLD).

6. Composition pharmaceutique selon l'une quelconque des revendications 1 à 3, pour une utilisation dans la prévention et/ou le traitement d'une stéatohépatite non alcoolique (NASH).

7. Composition pharmaceutique pour une utilisation selon la revendication 5 ou 6, dans laquelle la composition pharmaceutique est destinée à être administrée par voie orale, et les premier et deuxième composants sont destinés à être administrés en même temps ou en séquence à des moments différents.

8. Composition pharmaceutique pour une utilisation selon l'une quelconque des revendications 5 à 7, dans laquelle la composition pharmaceutique est destinée à être administrée à une dose de 1 à 1000 mg par jour.
